# EUROPEAN PATENT SPECIFICATION

(11) **EP 1 200 563 B1**
(45) Date of publication and mention of the grant of the patent: **02.11.2005**
(21) Application number: 00951687.3
(22) Date of filing: 28.07.2000
(51) Int. Cl.: C12N 7/04, C12N 7/01, C12N 15/86, C12N 5/10, A61K 39/145, A61K 39/295, A61K 35/76, A61K 48/00

(54) **ATTENUATED INFLUENZA VIRUS USEFUL AS VACCINE**
ALS IMPFSTOFF VERWENDBARES ATTENUIERTES INFLUENZAVIRUS
VIRUS ATTENUE DE LA GRIPPE UTILISABLE COMME VACCIN

(30) Priority: 30.07.1999 GB 9917981; 30.07.1999 US 146145 P
(43) Date of publication of application: 02.05.2002
(73) Proprietor: ISIS INNOVATION LIMITED, Summertown, Oxford OX2 7SG (GB)
(72) Inventor: BROWNLEE, George, Gow, Oxford OX1 3RE (GB); FODOR, Ervin Sir William Dunn School of Pathology, Oxford OX1 3RE (GB); POON, Leo Sir William Dunn School of Pathology, Oxford OX1 3RE (GB)
(74) Representative: Roques, Sarah Elizabeth
(86) International application number: PCT/GB2000/002933
(87) International publication number: WO 2001/009291

(56) References cited:
- WO-A-91/03552
- MANDL C. W. ET AL.: "Spontaneous and engineered deletions in the 3' noncoding region of tick-borne encephalitis virus: Construction of highly attenuated mutants of a flavivirus." JOURNAL OF VIROLOGY, vol. 72, no. 3, March 1998 (1998-03), pages 2132-2140, XP002151482 ISSN: 0022-538X
- LUO G. ET AL.: "Mechanism of attenuation of a chimeric influenza A/B transfectant virus." JOURNAL OF VIROLOGY, vol. 66, no. 8, 1992, pages 4679-4685, XP000953341 ISSN: 0027-8424
- FODOR E. ET AL.: "Attenuation of influenza A virus mRNA levels by promoter mutations." JOURNAL OF VIROLOGY, vol. 72, no. 8, August 1998 (1998-08), pages 6283-6290, XP002151484 ISSN: 0022-538X cited in the application
- POON L. L. M. ET AL.: "Direct evidence that the poly(A) tail of influenza A virus mRNA is synthesized by reiterative copying of a U track in the virion RNA template." JOURNAL OF VIROLOGY, vol. 73, no. 4, April 1999 (1999-04), pages 3473-3476, XP002151485 ISSN: 0022-538X cited in the application
- POON L. L. M ET AL.: "Polyuridylated mRNA synthesized by a recombinant influenza virus is defective in nuclear export." JOURNAL OF VIROLOGY, vol. 74, no. 1, January 2000 (2000-01), pages 418-427, XP002151486 ISSN: 0022-538X
- FODOR E. ET AL.: "Rescue of influenza A virus from recombinant DNA." JOURNAL OF VIROLOGY, vol. 73, no. 11, November 1999 (1999-11), pages 9679-9682, XP002151487 ISSN: 0022-538X

## Description

The present invention relates to modified viruses which can be used in a vaccine. Modified viruses of the invention also include recombinant attenuated viruses suitable for use as viral vectors for expression of heterologous sequences in target cells.

Attenuated viruses can be used to immunise against the diseases caused by the equivalent wild-type viruses. Vaccines made from attenuated viruses are often superior to vaccines containing dead virus or viral proteins produced by recombinant techniques. For example in the case of influenza present inactivated influenza virus vaccines provide only limited protection. Previous efforts to provide a safe, live attenuated influenza vaccine have focussed primarily on cold-adapted influenza viruses. Thus, attenuated influenza viruses have previously been obtained by extensively passaging influenza virus at low temperatures. As a result of adaptation to growth at low temperature, influenza viruses which have lost their ability to replicate at higher temperatures (about 39°C) are obtained. The replication of such cold-adapted (CA) viruses is only slightly restricted in the cooler upper respiratory tract, but highly restricted in the warmer lower respiratory tract, the major site of disease-associated pathology.

Sequence comparisons between wild-type and CA influenza viruses have revealed both silent mutations and non-silent mutations leading to amino acid changes predominantly in the coding regions of gene segments. Most amino acid changes were found to be the result of point mutations. The genetic instability of point mutations, and the level of immunogenicity of CA influenza viruses, remain as perceived potential problems in use of CA influenza viruses as vaccines for worldwide general use.

Genetic engineering may be used to modify viruses in order to attenuate them. The modification must weaken the virus without rendering it incapable of infecting host cells. The inventors have identified such a modification which affects the way that mRNA is processed in host cells, and therefore can be used for any negative-sense single stranded RNA virus that uses a similar polyadenylation strategy, and in particular influenza viruses. They have found that when a poly U track in a viral gene is replaced with a poly A track functional mRNA is still transcribed from the gene. Viruses with such modifications have been found to be attenuated. The modifications are also stable as point mutations in the poly A track disrupt transcription from the gene.

Mandl *et al* (1998) J. Virology 72, 2132-40 discloses spontaneous and engineered deletions in the 3' noncoding region of tick-borne encephalitis virus.

Luo *et al* (1992) J. Virology 66, 4679-85 discloses the mechanism of attenuation of a chimeric influenza A/B transfectant virus.

Fodor *et al* (1998) J. Virology 72, 6283-90 discloses attenuation of influenza A virus mRNA levels by promoter mutations.

WO 91/03552 discloses recombinant negative strand virus RNA templates which may be used to express heterologous gene products and/or to construct chimeric viruses.

Poon *et al* (1) discloses that poly A track in an influenza vRNA segment can be copied to provide a poly U tail for mRNA transcribed from the segment. However, this paper does not describe the characteristics of such mRNA, or suggest that the replacement of a poly U track in the genomic nucleic acid of a virus can be used to produce a functional attenuated virus.

The invention provides an attenuated negative-sense single stranded influenza virus comprising genomic RNA segments, at least one of which is altered by replacement of the poly U track thereof, located at the polyadenylation site near the 5'-end of the genomic RNA, by a poly A track which is capable of being copied to provide a 3'-poly U tail for mRNA transcribed from the genomic RNA segment.

The virus may have one or more genomic segments. A negative stranded RNA virus with several genomic segments is exemplified by influenza virus (1). The influenza virus may be influenza A, B or C.

In one embodiment the virus is one in which the poly A track is copied by a reiterative mechanism. Generally in such a mechanism the polymerase repeatedly "slips back" when transcribing the poly A track allowing formation of a poly U tail in the mRNA which is no longer than the poly A track. Influenza viruses use such a reiterative mechanism, and of these influenza A virus is particularly preferred.

The virus is attenuated and therefore will cause reduced disease symptoms (compared to the wild-type virus) when it is administered to a host. Thus one way of assessing attenuation is by measuring pathogenicity in a mouse model (e.g. as described in Example 7). Generally, the virus of the invention has a reduced ability to replicate in any, some or all of its natural host cells or in cell lines which the virus is able to infect. One way this can be measured is the reduction in virus titre when the virus is used to infect such cells. Typically the virus will exhibit at least a 0.3 log reduction, for example at least a 0.5 log, 0.8 log, 1 log ; 2 log , 5 log, 10 log or more reduction in titre. Generally the virus will be able to replicate to some degree in the host cell, i.e. be able to produce infectious daughter viruses.

Madin-Darby bovine kidney (MDBK) cells, Madin-Darby canine kidney cells or Vero cells can be used to measure the reduced ability of the virus to replicate.

Generally in the nucleic acid all of the uridines in the poly U track which can be copied to form the poly A tail are replaced by the same number, a reduced number or an increased (e.g. 1, 2, 3 or more fewer or greater) adenosines. Typically the entire U₆ track is replaced by an A₅, A₆, A₇, A₈, A₉, A₁₀ track or a track comprising more than 10 adenosines such as 11 to 15 adenosines or more.

Typically the nucleic acid will differ from the equivalent wild-type nucleic acid by 4, 5, 6, 7 to 10, 10 to 20 or more mutations (which can be insertions, deletions or substitutions). Such mutations may typically be at the 5' and/or 3' terminus. Typically the nucleic acid is homologous to and/or capable of hybridising to the equivalent wild-type genomic segment. In one embodiment the nucleic acid is a chimera of fragments from different viruses. The nucleic acid may be a chimera of fragments from an influenza A and/or B and/or C virus.

The nucleic acid is capable of being expressed in a host cell to produce at least one mRNA with a poly U tail. Generally proteins encoded by the nucleic acid (and by this mRNA) are expressed at reduced levels in the cell (compared to the equivalent mRNA with a poly A tail). At least one of the proteins is one which causes attenuation when expressed at levels below the level at which they are expressed by the wild-type virus.

The proteins are typically wild-type proteins or functional equivalents which have at least some of the activity of the wild-type proteins. The proteins are typically structural proteins (e.g. a coat protein), non-structural proteins or enzymes (e.g. a polymerase).

The virus may contain 1, 2, 3, 4 or more nucleic acids in which the poly U track has been replaced by a poly A track. The nucleic acid is typically a mutated native influenza virus genomic segment. This segment may encode the influenza proteins: PB1, PB2 or PA polymerase proteins, the nucleoprotein (NP), the matrix proteins M1 or M2, haemagglutinin (HA), neuraminidase (NA), NS1 or NS2; or functional equivalents of any of these proteins. Preferably the segment encodes NA.

A virus of the invention may additionally contain a heterologous coding sequence capable of being expressed in host cells. Such a heterologous coding sequence may encode an antigenic peptide or polypeptide capable of stimulating an immune response (either an antibody response or a cell-mediated immune response) to a pathogenic agent. Representative examples of such pathogenic agents are viruses, e.g. other influenza viruses or non-influenza viruses such as HIV, bacteria, fungi, parasites, eg. malaria parasites, and disease-causing cells such as cancer cells.

For vaccinating purpose, a heterologous coding sequence may be provided in an attenuated virus of the invention encoding an antigen of a pathogenic agent or a modification thereof capable of stimulating an immune response. The heterologous coding sequence may be inserted into a viral gene to provide a fusion protein which retains the function of the parent viral protein. In the case of influenza virus one site which has previously been found to tolerate insertions of foreign antigens (epitope grafting) is the antigenic B site of HA. Antigenic site B of that surface protein consists of an exposed loop structure located on top of the protein and is known to be highly immunogenic. Manipulation of the HA gene of an influenza virus to insert a viral epitope in the HA protein B site has previously been reported (see again the studies of Muster *et al*. reported in 2 and the studies of Li *et al*. reported in 3). The same strategy has also previously been employed by Rodrigues *et al*. to express B-cell epitopes derived from a malaria parasite (4). Heterologous coding sequences for an antigenic polypeptide may also, for example, be preferably inserted into an influenza virus NA gene. Strategies for epitope grafting into influenza viral proteins have also previously been described, for example, in WO91/03552.

Epitope grafting of a foreign sequence into an influenza virus protein may result in a non-functional chimeric viral protein and make the rescue of a viable transfectant virus impossible. A different strategy for expressing foreign sequences by recombinant influenza viruses, which may be applied to attenuated viruses of the present invention, involves the engineering of gene segments containing an additional open reading frame. A recombinant genomic segment may be constructed which provides an internal ribosome entry site for a heterologous coding sequence. This approach has previously been used, for example by Garcia-Sastre *et al*. to obtain an influenza virus vRNA segment which encodes both a truncated form of gp41 of HIV and NA (5). Alternatively, a heterologous coding sequence may be fused in frame to a viral protein coding sequence to encode a chimeric polyprotein capable of autoproteolytic protease cleavage to give the viral protein and a desired second polypeptide, e.g. a viral antigen. This strategy has been shown by Percy *et al*. to be suitable for expressing non-influenza proteins up to 200 amino acids in length (6).

It will be appreciated that a recombinant attenuated virus of the invention may be employed as a vehicle for expression of heterologous coding sequences in target cells for a variety of therapeutic purposes in addition to vaccination. Such a recombinant virus may, for example, have a genomic segment encoding any of the following:
- a cytokine such as an interferon or an interleukin,
- a toxin,
- a palliative capable of inhibiting a function of a pathogenic agent either directly or indirectly, e.g. a viral protease inhibitor
- an enzyme capable of converting a compound with little or no cytotoxicity to a cytotoxic compound, e.g. a viral enzyme such as Herpes simplex thymidine kinase capable of phosphorylating purine and pyrimidine analogues to active toxic forms,
- an antisense sequence,
- a ribozyme.

Sequences encoding such agents may be incorporated into an attenuated virus of the invention by any of the techniques previously referred to above in connection with providing attenuated viruses of the invention expressing foreign epitopes. A recombinant virus of the present invention may be employed for gene therapy.

The nucleic acid in which the poly U track has been replaced invention may be complexed *in vitro* with influenza viral polymerase proteins and nucleoprotein to form a RNP complex which has all the components necessary for transcription and replication. Such RNP complexes, which constitute a still further aspect of the present invention, may be prepared in conventional manner as previously employed for incorporation of genetically-engineered influenza virus RNA genomic segments into RNA complexes for viral rescue in cells (7, 8, 9). RNP complexes of the invention may be transfected into cultured cells, e.g. MDBK cells, MDCK cells or Vero cells, again using conventional techniques. Methods commonly employed for this purpose include DEAE-dextran transfection and electroporation (10, 11).

The invention also provides an *ex vivo* cell infected by a virus of the invention. Such cells includes human and/or animals cells normally infected by influenza viruses. The cells may be of a single cell type or more than one type, e.g. cultured human or non-human animal cells of one or more than one type. They may be *in vivo* cells, e.g. cells of an animal model. The cells may be any of the types of cells mentioned below in relation to selection systems. These types of cells can be used in the method of making the virus described below.

The virus of the invention may be made in a method comprising providing in a host cell the nucleic acid of the invention, and any other genomic nucleic acids which are required (for the virus to be viable) under conditions whereby the nucleic acids are packaged into a viral particle. Such conditions typically include the presence of the proteins required to form the viral particle. The genomic nucleic acids may be provided in the host cell by plasmids.

RNP complexes of the invention as hereinbefore described may be transfected into host cells that have previously been infected with an influenza helper virus to complement the RNP complexes and enable selection of the desired attenuated viral particles. A number of helper virus-based cellular rescue systems for particular influenza virus genes have previously been described and have been reviewed by Muster and Garcia-Sastre (12). Such gene specific rescue systems are briefly summarized below.

### Helper virus based influenza gene rescue systems

Helper based rescue systems have been reported allowing the genetic manipulation of influenza A vRNAs for NA and HA surface antigens, the non-structural proteins, NP, PB2 polymerase protein and the M proteins.

### NA gene specific rescue system

The most commonly employed helper virus based influenza gene rescue system is limited to the NA of influenza A/WSN/33 virus (7, 8). This method is based on the observation that only influenza viruses with an NA gene from influenza A/WSN/33 are able to grow on MDBK cells in the absence of trypsin. In this rescue system, the helper virus is a reassortant containing seven gene segments from influenza A/WSN/33 and a NA gene from a virus other than influenza A/WSN/33. Generally A/WSN-HK, which has an NA gene from influenza A/HK/8/68, is used as the helper virus. In this system, the NA gene of influenza A/WSN/33 is transfected into cells infected with the helper virus. The virus is then selected by growing on MDBK cells in the absence of exogenous proteases.

NA genes can also be rescued by using a NA-deficient mutant virus as a helper virus. Such a helper virus requires exogenous neuraminidase to grow in tissue culture. The NA-gene is transfected into cells infected with the helper virus. The virus is then selected by growing on cells in the absence of neuraminidase (13).

### NS gene specific rescue system

A temperature-sensitive influenza virus with a defect in the NS1 protein is used as the helper virus of a NS gene specific rescue system. The NS gene segment carries two overlapping genes coding for the NS1 and NS2 proteins. This rescue system allows the rescue of a NS gene segment encoding an NS1 protein which has activity at the non-permissive temperature. In this system, the NS gene segment which is to be rescued is transfected into cells infected with the temperature-sensitive virus. The virus with the transfected NS gene segment is selected by growing the virus at the non-permissive temperature as described by Enami *et al*. (14) and Egorov *et al* (26).

### PB2 gene specific rescue system

A virus with an avian influenza A virus PB2 gene can be used as the helper virus in a PB2 gene specific rescue system. The avian influenza A virus PB2 gene restricts the replication of the helper virus in mammalian cells. Therefore, this rescue system can rescue a PB2 gene which allows replication of influenza virus in mammalian cells. The PB2 gene which is to be rescued is transfected into cells infected with the helper virus..The virus with the transfected PB2 gene is selected by growing the virus in mammalian cells. Subbarao *et al*. (15) have used such an avian influenza A virus PB2 gene based system to rescue the PB2 gene of wild-type influenza A/Ann Arbor/6/60 virus.

### M gene specific rescue system

An amantidine-sensitive influenza virus carrying an M gene of influenza A/equine/Miami/1/63 virus can be used as a helper virus of an M gene specific rescue system. The rescue system allows the rescue of an M gene which confers amantidine resistance to a virus. In this system, the M gene which is to be rescued is transfected into cells infected with the helper virus. The virus with the transfected M gene is selected by growing the virus in the presence of amantidine. Castrucci and Kawaoka (16) have used such an amantidine-sensitive M gene based system to rescue the M gene of influenza A/PR/8/34 virus.

### Antibody-based rescue systems

These systems depend on the binding or non-binding of the transfectant virus to a particular antibody (8, 17). Such antibody is a neutralising antibody which binds to influenza virus and impairs its growth in tissue culture. The helper virus may, for example, carry a gene which encodes an influenza surface protein which displays the antibody epitope. This system can therefore be used to select for transfectant virus which does not carry such a gene, but which of course is viable. This type of rescue system thus allows the rescue of a gene encoding an influenza surface protein. The gene to be rescued is transfected into cells infected with the helper virus. The virus with the transfected gene is selected by growing the virus in the presence of the antibody. Such a system was used by Enami and Palese (8) to rescue a transfected synthetic HA segment.

### NP gene specific rescue system

Li and coworkers (11) reported a reverse genetics system for the rescue of the influenza A virus nucleoprotein gene. In this system, a temperature-sensistive (ts) mutant ts56 is used as a helper virus. RNA complexes are reconstituted *in vivo* as described before (8) and are then introduced by electroporation into ts56 helper virus infected cells. Transfectant viruses with a rescued NP-encoding vRNA segment are selected at the non-permissive temperature by plaqueing on MDBK cells.

### Influenza B virus rescue system

Barclay and Palese (18) have additionally described the rescue of HA genes in an influenza B virus.

The preparation of an attenuated influenza virus of the invention may alternatively be achieved using the expression vector-based influenza gene rescue strategy developed by Pleschka *et al*. (19). In contrast to the RNP transfection system referred to above, this eliminates the need for purification of the viral NP and polymerase proteins which is required for *in vitro* reconstitution of RNP complexes. Expression vectors are co-transfected into host cells which will provide the NP and P proteins (i.e. PB1, PB2 and PA) and also a nucleic acid of the invention. In this case, RNP complexes of the invention are formed intracellularly. The cells may then be infected with an influenza helper virus as previously described to select for the required attenuated influenza virus .

An RNA complex of the invention may also be rescued in host cells into a viable attenuated virus by transfecting into the host cells additional complementing RNA complexes thereby eliminating the need for a helper virus. This may be achieved in accordance with the general rescue strategy for influenza virus genes more recently described by Enami (20). This strategy involves purifying RNPs from an appropriate influenza virus and treating the RNPs *in vitro* with RNase H in the presence of a cDNA which hybridizes to the influenza virus gene to be rescued. In this way specific digestion of that gene by the RNase H is achieved. The gene depleted RNPs are then co-transfected into cells with the RNP-complex containing the nucleic acid of the invention. The cells are then overlaid with agar and transfectant attenuated viruses obtained by direct plaque formation. This strategy, unlike the above described helper virus-based gene rescue strategies, can be applied to any influenza gene from any influenza virus. It can thus be applied to obtain an attenuated virus or nuclec acid of the invention of any influenza type.

Helper-independent methods of viral rescue may also be used. Generally such methods are based on cells capable of expressing the complete genomic vRNA segments of an influenza virus, or the corresponding cRNAs. Typically such cells are capable of providing a nucleoprotein and RNA dependent RNA polymerase whereby RNP complexes containing the genomic vRNA segments can be formed and viral particles can be assembled with the cells. Suitable systems are described in (27) and (28).

From the discussion of the properties of the virus of the invention above it will be appreciated that the virus of the invention is suited for use in immunisation. Thus, in yet another aspect, the present invention provides a vaccine comprising a virus of the invention. Particularly preferred are such vaccines wherein the virus acts as a combined vaccinating agent against more than one pathogenic agent, e.g. the wild-type form of the virus of the invention and a second pathogenic agent other than the wild-type form of the virus of the invention. Such vaccines may be formulated and administered in accordance with known methods for this purpose.

Thus in a still further aspect, the present invention provides a method of stimulating an immune response against a virus, e.g. an influenza virus, either alone or together with stimulation of an immune response against one or more further pathogenic agents, which comprises administering in an immunising mode a virus of the invention capable of inducing said immune response(s). In the case of influenza intranasal immunisation with virus of the invention may, for example, be preferred. Such immunisation may be carried out as illustrated by the immunisation studies with recombinant influenza viruses expressing an HIV-epitope reported by Muster *et al*. (2) and Ferko *et al*. (21). A suitable immunisation dose may be, for example, in the range of 10³-10⁹ pfu. Booster immunisations may be given following an initial immunisation with a virus having the same functional characteristics, but of a different subtype or type.

Methods for incorporating heterologous coding sequences into an influenza virus have previously been described, for example, in Published International Application WO91/03552 (Palese *et al*.) and are also reviewed by Muster and García-Sastre in Textbook of Influenza 1998 (12). The heterologous coding sequence may be on the nucleic acid of the invention or on a different genomic segment. It may be carried by an additional nucleic acid segment also incorporating a gene for an influenza viral protein to provide selection pressure. It has previously been reported, for example, that an influenza virus can be constructed carrying at least 9 different vRNA segments (14).

Use of attenuated recombinant influenza viruses of the invention as vectors to express foreign antigens for vaccinating purposes is an attractive therapeutic strategy since:
1. Antibodies to the different subtypes show little cross-reactivity. One drawback with the use of a virus as a vaccine is that an immune response will be produced to the virus. It is often desired that one or more booster immunisations comprising the same antigen are given after the initial immunisation. However, the immune response to the virus reduces the effectiveness of subsequent immunisations with the samevirus. Since antibodies to different influenza subtypes show little cross-reactivity, subsequent immunisations with an influenza virus of a different subtype but which expresses the same antigen should overcome this effect.
2. Influenza viruses have been shown to induce strong cellular and humoral responses.
3. Influenza viruses have been shown to induce strong mucosal responses. Intranasal immunisation with influenza virus has been shown to induce long lasting responses in genital and intestinal mucosa.
4. Influenza viruses are non-integrating and non-oncogenic.
5. As previously noted above, attenuated influenza viruses of the invention can be anticipated to be attenuation stable.

A recombinant virus of the invention may be administered directly or used to infect cells *ex vivo* which are then administered to a patient.

Thus, in still further aspects, the present invention provides a pharmaceutical composition comprising a recombinant virus of the invention in combination with a pharmaceutically acceptable carrier or diluent for delivery of a heterologous coding sequence to target cells. It also provides *ex vivo* cells infected by a virus of the invention and such cells hosting a virus of the invention formulated for administration with a pharmaceutically acceptable carrier or diluent. In yet another aspect, the present invention provides a method of delivering a heterologous coding sequence to cells which comprises infecting said cells with a virus of the invention carrying said sequence.

Viruses of the invention may also find use as a helper virus to rescue genes which can substitute for the gene(s) affected by the attenuating mutation(s) to provide viruses showing increased growth on a selected cell type. For this purpose, an attenuated virus will preferably be chosen which exhibits at least about a 3-4 log, preferably at least about a 5 log, reduction in growth compared to the corresponding wild-type virus on one or more cell types. Thus, in yet another embodiment, the present invention provides use of a virus of the invention as a helper virus to rescue nucleic acid in cells, wherein viruses produced containing said segment are selected on the basis of increased growth compared with the helper virus on cells of a selected type. For example, an influenza A virus of the invention having an attenuating poly U track replacement in NA-encoding vRNA may be usefully employed to rescue an NA-encoding vRNA or functional modification thereof derived from a second influenza A virus. A typical protocol for this purpose will comprise the steps of:
1. infecting cells with the helper virus,
2. transfection of an RNP complex containing the gene(s) to be rescued into the helper virus infected cells, and
3. selection of rescued viruses, either on the same cell type or a different cell type on which the helper virus shows increased attenuation.
The cell type in step 3 will be chosen such that only viruses which have acquired the transfected gene(s) are expected to grow to high titre. Cultured cells which may prove useful in the selection of attenuated viruses of the invention *in vitro* include one or more of MDBK cells, Madin-Darby canine kidney (MDCK) cells and Vero (African green monkey kidney) cells.

A virus of the invention which is an influenza A/WSN/33 virus is particularly favoured as a helper virus for use to rescue NA genes originating from other influenza viruses of the A-type. In this case, MDBK cells may, for example, be initially infected with such a helper virus and Vero cells preferably used for selection of viruses carrying an NA gene containing vRNA without an attenuating mutation.

Influenza A/WSN/33 is known to exhibit in mice neurovirulence associated with the surface antigen NA. For this reason attenuated modified versions of that virus are not regarded as suitable for direct vaccine use. However, when used as a helper virus as above, NA vRNAs may be obtained for site-directed mutagenesis to construct alternative attenuated influenza A viruses according to the invention more suitable for therapeutic, e.g. vaccine, use.

The invention is further illustrated by the accompanying drawings in which:

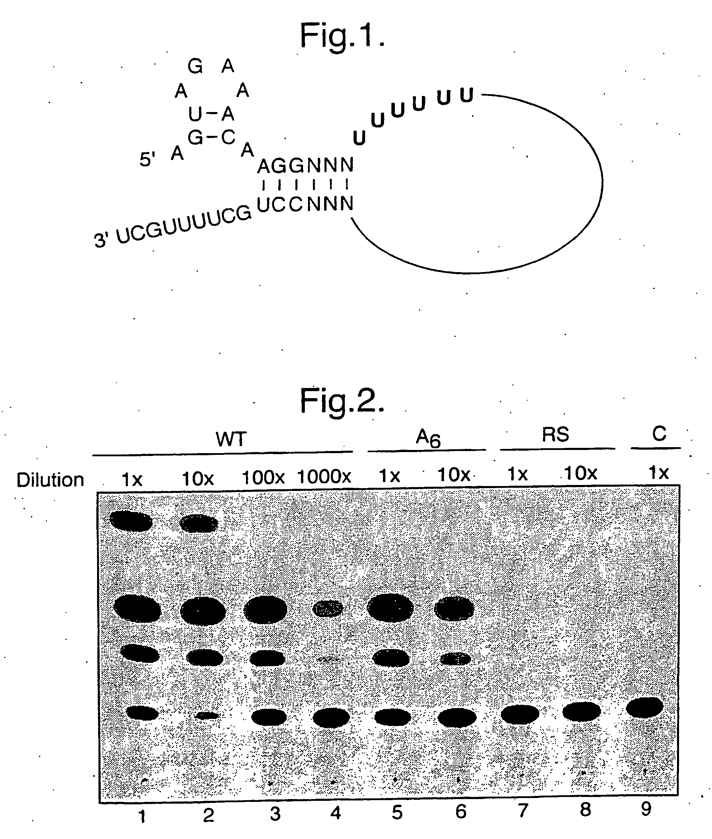

Figure 1 shows a model of vRNA with the wild-type conserved terminal sequences. The proposed RNA hook model of the vRNA template is shown. The U₆ track (the polyadenylation site) is shown in bold.
Figure 2 shows the effects of mutations of the U₆ track on CAT expression. Human 293 kidney cells were tranfected with 4 protein expression plasmids (pGT-h-PB1, pGT-h-PB2, pGT-h-PA and pGT-h-NP) and pPOL-I-CAT-RT with a wild-type polyadenylation signal (lanes 1 to 4), an U₆→A₆ mutation (A₆; lanes 5 and 6), or a U₆→ UCUACG mutation (RS; lanes 7 and 8). The CAT activities of the cell lysates were analyzed as described in Materials and Methods. Dilution factors of the cell lysates are indicated. Lane 9, mock transfection (C).

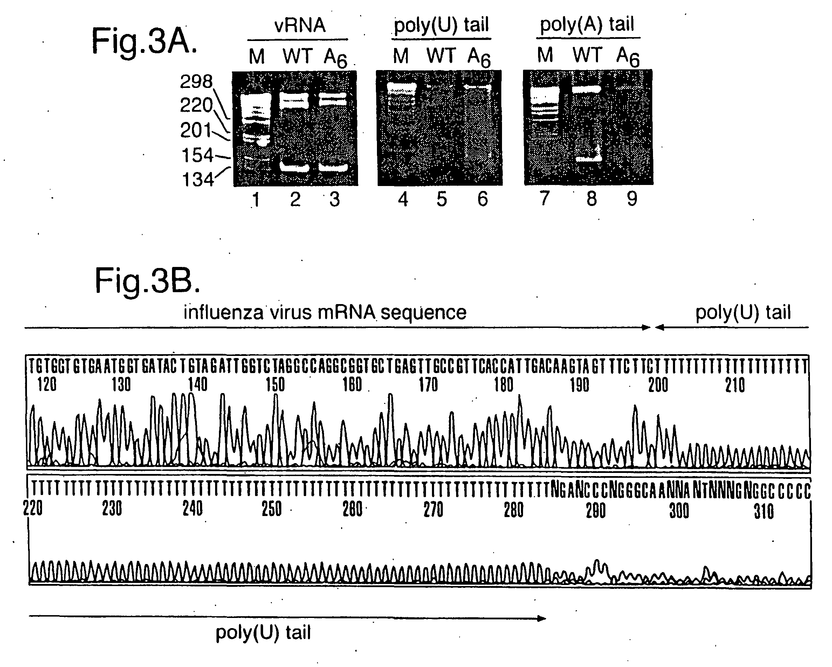

Figure 3 shows that poly(U)-tailed NA mRNA is synthesized by the A₆ transfectant virus. (A) RT-PCR assays. Total RNA from cells infected with the wild-type virus (WT; lanes 2, 5 and 8) or the A₆ mutant (A₆; lane 3, 5 and 9) was tested by RT-PCR reactions for vRNA (lanes 2 and 3), poly(U)-tailed NA mRNA (lanes 5 and 6) and poly(A)-tailed NA mRNA (lanes 8 and 9). RT-PCR products were analyzed on 8% native acrylamide gels. The high molecular weight bands near the wells are non-specific PCR products. Lanes 1, 4 and 7 are DNA markers (M). (B) Poly(U) sequence (n=84) of a cDNA clone derived from poly(U)-tailed NA mRNA. The influenza virus mRNA sequence is indicated.

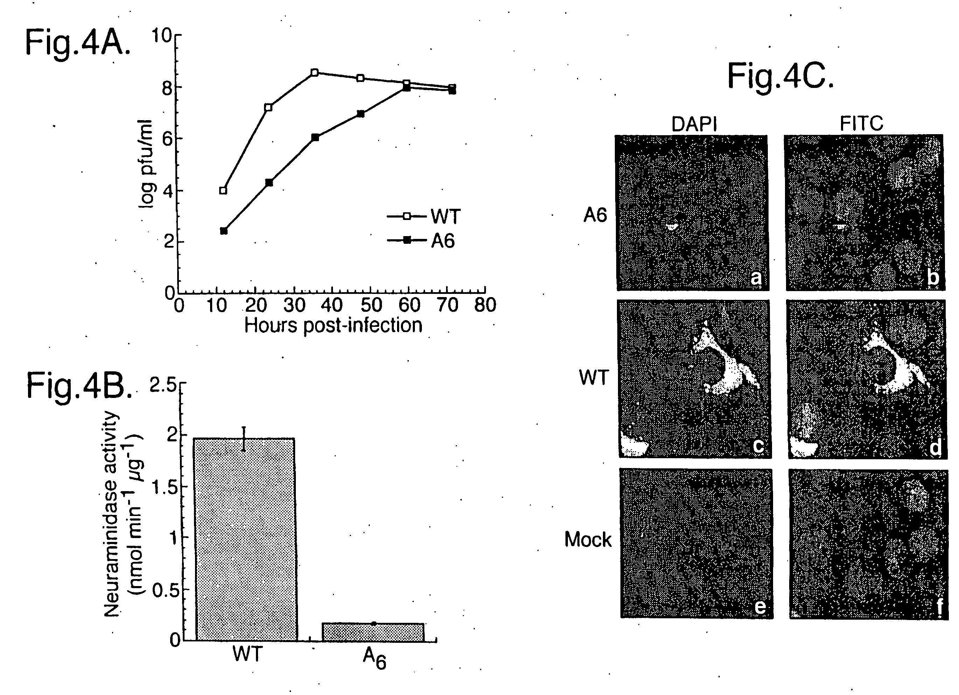

Figure 4 shows that the A₆ mutant is attenuated due to the reduction of NA expression (A) Growth curves of transfectant viruses on MDBK cells: Cells were infected with the wild-type virus (WT) or the A₆ mutant (A₆) at 0.01 MOI. Infectious particles released into the media at the indicated time points were titrated by plaque assay in MDBK cells. (B) NA activities of wild-type virus (WT) and the A₆ mutant (A₆). NA activity was expressed as nanomoles of 4-methylumbelliferone released per min per microgram of viral protein. (C) NA protein expression in infected cells. Cells infected with A₆ mutant (panel a and b), wild-type virus (panels c and d) and mock-infected cells (panels e and f) were tested for NA protein expression. NA protein expression was visualized by fluorescence microscopy using of FITC-labelled antibody. Cells were mounted in antibleach medium containing 4',6-diamidino-2-phenylindole (DAPI) for DNA staining. The second column of the panels is the merged image of NA protein expression and cell nuclei. Note that not all cells were infected with virus.

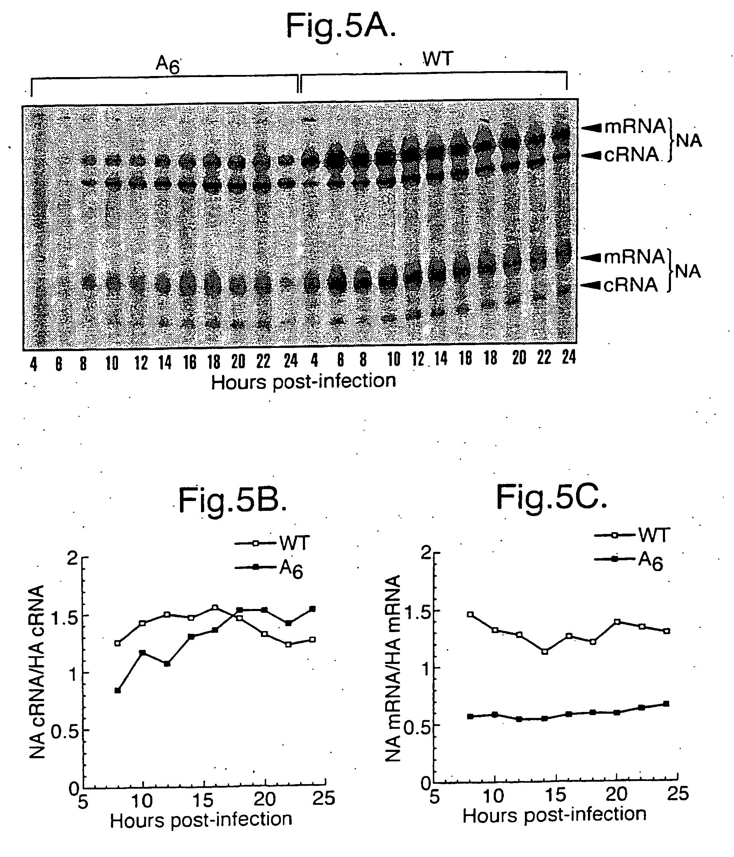

Figure 5 shows a primer extension assay for NA-specific vRNA. (A) NA-specific vRNA levels in cells infected with the A₆ mutant (A₆) and the wild-type virus (WT). Total RNAs from infected cells were isolated at the indicated time points p.i. The expected products for NA (259 nt) and NS (196 nt) vRNA are indicated. (B) NA-specific vRNA levels in purified A₆ mutant (A₆) and wild-type virus (WT). The expected products for NA (259 nt) and NS (196 nt) vRNA are indicated. (C) NA vRNA to NS vRNA ratio in cells infected with the A₆ mutant (A₆) or wild-type virus (WT).

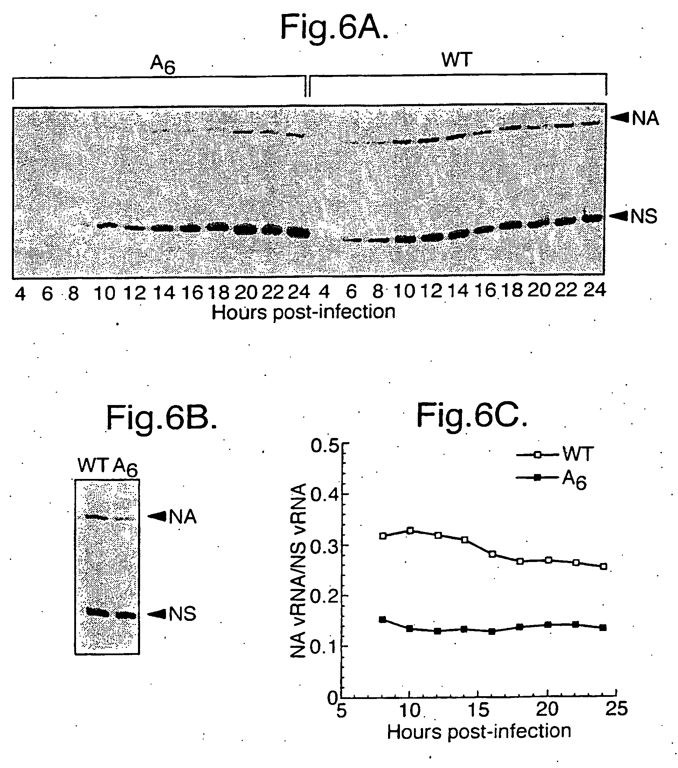

Figure 6 shows a primer extension assay for NA-specific cRNA and mRNA. (A) NA-specific cRNA and mRNA levels in cells infected with the A₆ mutant (A₆) and the wild-type virus (WT). Total RNA from infected cells was isolated at the indicated time points p.i. The expected products for NA cRNA (142 nt), NA mRNA (152-157 nt), HA cRNA (94 nt) and HA mRNA (104-109 nt) are indicated. Since influenza virus mRNAs are initiated by capped RNA primers, which are heterogeneous in size, primer extension products of the mRNA are expected to produce a broad band which is 10-15 nt longer than products of cRNA. (B) NA cRNA to HA cRNA ratio in cells infected with the A₆ mutant (A₆) or the wild-type virus (WT). (C) NA mRNA to HA mRNA ratio in cells infected the A₆ mutant (A₆) or the wild-type virus (WT).

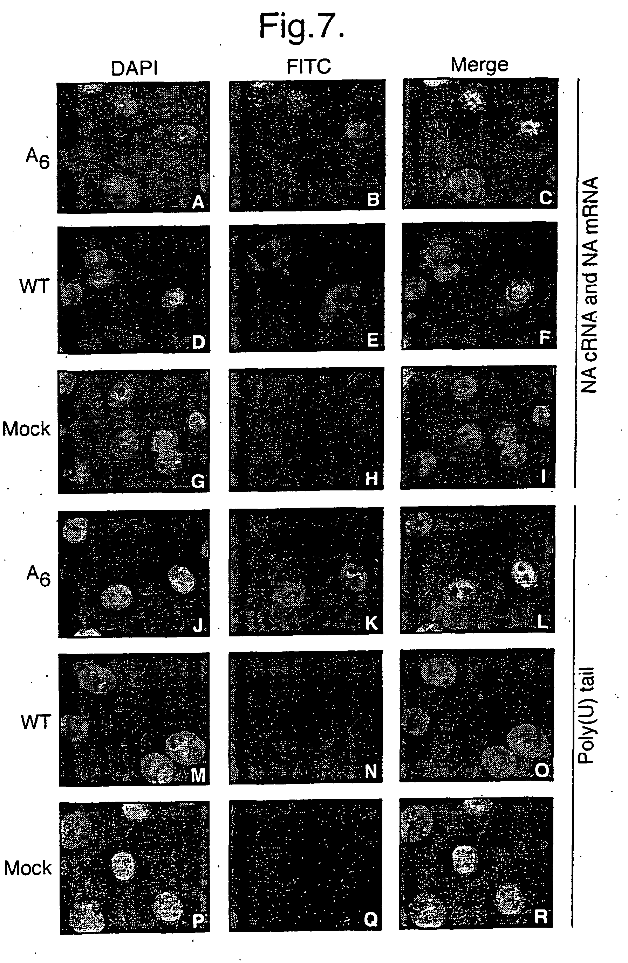

Figure 7 shows that poly(U)-tailed NA mRNA is predominantly localized in the nucleus. Cells infected with the A₆ mutant (panels A-C and J-L), wild-type (panels D-F and M-O) virus and mock-infected cells (panels, G-I and P-R) were hybridized with NA mRNA-specific (panels A-I) or poly(U)-tailed NA mRNA-specific (panels J-R) probes. Nuclei of cells were stained with DAPI (first column). The distribution of corresponding probe was visualized by fluorescence microscope (FITC; second column). The third column (Merge) is the merged image of the first and second columns. Note that not all the cells were infected with virus.

The following examples also illustrate the invention.

### Example 1

### The effect on expression of replacing the poly U track of influenza virus

Replacing the U-track of influenza virus (Fig. 1) with an A-track in a model vRNA template resulted in synthesis of poly(U)-tailed mRNA both *in vitro* and *in vivo*. To test whether a poly(U)-tailed mRNA could be used for gene expression, we used a plasmid-based chloramphenicol acetyltransferase (CAT) reporter system. A CAT vRNA expression plasmid, pPOLI-CAT-RT (19), was transfected into human 293 kidney cells to synthesize a vRNA-like template containing a CAT gene in negative orientation, flanked by the 5' and 3' non-coding regions of segment 8 of influenza A/WSN/33 virus. Four protein expression plasmids which encode viral PB 1, PB2, PA and NP proteins were also cotransfected into the cell for the replication and transcription of the CAT vRNA template. To synthesize poly(U)-tailed mRNA, the polyadenylation site (U₆ track) in the 5' noncoding region of the CAT vRNA template was mutated into an A₆ track, The presence of poly(U) sequences at the 3' end of mRNAs transcribed from this mutant template has been rigorously demonstrated elsewhere (1).

When cells were transfected with a wild-type CAT vRNA expression plasmid, strong CAT activity was detected (Fig 2, lanes 1 to 4). However, when the U₆→A₆ construct (see above) was transfected into the cells, only low (3 ± 1.5% of the wild type) but significant CAT activity was detected (Fig 2, compare lanes 5 and 6 with lanes I to 4). By contrast, cells transfected with a control plasmid which contained a U₆→ UCUACG "random" mutation had no detectable CAT activity (Fig 2, lanes 7 and 8). These results confirm that a homopolymeric-track near the 5' end of vRNA was required for pausing of the polymerase, polymerase slippage and synthesis of a homopolymeric tail. Since no gene expression was detected when the U₆ track was mutated into a "random" sequence (Fig 2, lanes 7 and 8), these results also demonstrated that a homopolymeric track (either U₆ or A₆) is essential for gene expression.

### Example 2

### Rescue of an influenza virus encoding an NA-specific mRNA with a poly(U) tail

Since the poly(U)-tailed mRNA transcribed from the CAT vRNA was translated, though at low efficiency, it was of interest to test whether we could rescue a transfectant influenza virus which could synthesize poly(U)-tailed mRNA. We decided to replace the polyadenylation signal (U₆) with an A₆ sequence in the vRNA of the NA segment, partly because of the availability of an efficient rescue system for this gene segment (7) and partly because influenza virus is known to tolerate severe reduction in NA protein levels. To construct a transfectant virus with an A-track (hereafter called A₆ mutant) in the polyadenylation site of the NA segment, a synthetic NA vRNA containing a U₆→A₆ mutation in its 5' non-coding region was first reconstituted into ribonucleoproteins (RNPs). The reconstituted RNPs were then tranfected into cells infected with A/WSN/HK helper virus (8). In three independent transfections, both wild-type and A₆ mutant viruses were rescued. However, when the U-track was mutated into a random sequence (UCUACG), no transfectant virus could be rescued. The transfectant viruses were plaque purified three times and a single plaque of each virus was used for further analysis.

To confirm the presence of the U₆→A₆ mutation in the NA vRNA of the A₆ mutant, vRNA from purified virus was isolated, amplified by RT-PCR, cloned, and sequenced. Apart from the introduced U₆→A₆ mutation, no other mutation was detected in the 5' and 3' non-coding regions of this segment (data not shown) other than a mutation of G→C at residue 195 (shown in Fig 3B). This additional mutation was inadvertently introduced in the mutagenesis protocol for generating the U₆→A₆ mutant NA plasmid prior to reconstitution of RNP's. This additional mutation in the 5' noncoding non-conserved region of the NA segment is not expected to contribute to the properties of the U₆→A₆ mutant, as demonstrated in previous studies (see construct NA5D in (29)).

The U₆→A₆ mutation could be detected even after ten passages of the A₆ mutant on MDBK cells (data not shown), indicating that the U₆→A₆ mutation was stable at least over 10 passages.

### Example 3

### Poly(U)-tailed NA mRNA is detected in cells infected with the A₆ mutant

To confirm that the U₆→A₆ mutation in the NA vRNA resulted in the synthesis of poly(U)-tailed NA mRNA, we isolated total RNA from cells infected with the wild-type virus or the A₆ mutant. Total RNA was then tested by an RT-PCR assay which is specific for the detection of either poly(A)-tailed or poly(U)-tailed mRNA (22). In reverse transcription reactions, 5'-GC-clamped T₂₀ and 5'-GC-clamped A₂₀ primers were used to detected poly(A)-tailed and poly(U)-tailed mRNA, respectively. The resultant positive signal appears as a characteristic smear as described before (22). As shown in Fig. 3A, poly(U)-tailed NA mRNA was detected in the total RNA isolated from cells infected with the A₆ mutant (lane 6), but not in that isolated from cells infected with the wild-type virus (lane 5). By contrast, poly(A)-tailed NA mRNA could only be detected in RNA from cells infected with the wild-type virus (lane 8), but not in the RNA from cells infected with the A₆ mutant (lane 9). These results showed that poly(U)-tailed NA mRNA was specifically synthesized in cells infected with the A₆ mutant and it was not polyadenylated. Lanes 2 and 3 in Fig. 3A show RT-PCR products corresponding to the 5' end of NA vRNA isolated from cells infected with the wild type and the A₆ mutant, respectively. The strong signals comigrating with the 134 nucleotide (nt) DNA marker indicate that cells used for RNA isolation were, as expected, infected with viruses. The RT-PCR products from poly(U)-tailed mRNAs (lane 6) were then cloned and sequenced (see Materials and Methods). Nine independent clones were found to contain poly(U) sequences in the range of 40 to 127 nucleotides. Fig. 3B shows one example with a poly(U) tail of 84 U residues.

### Example 4

### The A₆ mutant is attenuated due to the reduction of NA protein expression

The A₆ mutant consistently produced small plaques when grown on Madin-Darby bovine kidney (MDBK) cells (data not shown), suggesting that this mutant was attenuated. To characterize the growth properties of the A₆ mutant in more detail, confluent MDBK cells were infected at multiplicity of infection (moi) of 0.01 and the number of infectious viral particles released into the medium was assayed by plaque assay on MDBK cells. As predicted from the plaque size, the A₆ mutant was attenuated. The maximum plaque titre of the A₆ mutant was about 1 log lower than that of the wild type (Fig. 4A). The A₆ mutant was also tested on Madin-Darby canine kidney (MDCK) cells and there was, again, about a 1 log reduction in maximum plaque titre compared to the wild-type virus (data not shown).

In the light of the CAT reporter gene study (see above, Fig. 2), the mostly likely explanation for the attenuation of the A₆ mutant was the reduction of NA protein expression. To test this possibility, a spectrofluorometric assay (see Materials and Methods) was used to analyse the NA activity associated with the purified virus. The NA activity of the A₆ mutant was about 8% of the wild type (Fig. 4B). We then used a monoclonal anti-NA antibody to show that the reduction of NA protein in virions was due to the low expression of the NA protein in infected cells. As shown in Fig. 4C, NA was highly expressed in the cytoplasm of the cells infected with the wild-type virus (panel d). By contrast, cells infected with the A₆ mutant expressed significantly less NA protein (panel b). Although the results of the immunofluorescence assay are not quantitative, they are in agreement with the results in Fig. 4B showing that reduced amounts of NA were incorporated into virions. In addition, these results suggest that the attenuation of the A₆ mutant was due to the reduction of the NA protein expression in cells infected with the A₆ mutant.

### Example 5

### Levels of the NA vRNA and mRNA. but not cRNA are specifically reduced

The above results showed that the NA protein expression in cells infected with the A₆ mutant was severely reduced. Such reduction could be due to low levels of poly(U)-tailed mRNA synthesis in infected cells. To test this hypothesis, total RNA was isolated from infected cells at different time points and the amounts of NA-specific vRNA, cRNA and mRNA were quantified by primer extension assays. In the primer extension assay for NA vRNA analysis, the NS vRNA was used as an internal control. As shown in Fig. 5A, at every time point tested, NA vRNA levels in cells infected with the A₆ mutant were lower than those in cells infected with the wild-type virus. By contrast, the NS vRNA levels from cells infected with the A₆ mutant were similar to those of the wild type. When the NA vRNA levels were normalized to the NS vRNA levels in infected cells, the steady state NA vRNA levels of the A₆ mutant were between 40-53 % of those of the wild type (Fig. 5C). We also quantified the amount of NA vRNA in virions (Fig. 5B). The levels of the NA vRNA in the transfectant virus (the A₆ mutant) were about 60 % of that of the wild type (Fig. 5B). Since both cRNA and mRNA are positive sense, the same NA-specific primer was used to analyze both these RNAs. In these primer extension reactions, a haemagglutinin (HA)-specific primer was used as an internal control. As shown in Fig 6A, cells infected with the wild-type virus had more NA mRNA then the NA cRNA. By contrast, the NA mRNA signals in cells infected with the A₆ mutant were much weaker than NA cRNA signals. These results suggested poly(U)-tailed mRNA was synthesized in a reduced level. When the levels of the NA cRNA were normalized to the internal control (i.e. HA cRNA), the steady state cRNA levels of the A₆ mutant were not statistically different from those of the wild type in a student t-test (from 3 independent experiments) (Fig. 6B). By contrast, when we normalized the NA mRNA to the internal control (i.e. HA mRNA), the steady state mRNA levels of the A₆ mutant in infected cells were only about 40 to 50% of those of the wild type (Fig. 6C). These results demonstrated that the NA mRNA, but not the NA cRNA of the A₆ mutant was synthesized at reduced levels.

### Example 6

### Poly(U)-tailed NA mRNA is predominantly localized in nucleus

Although cells infected with the A₆ mutant showed a reduction of the NA mRNA levels, such reduction could not fully explain the dramatic reduction of the NA activity of the A₆ mutant (8% of the wild type; Fig 4B). Since the influenza virus mRNAs are synthesized in the nucleus of infected cells, it was possible that the poly(U)-tailed NA mRNA was defective in nuclear export. To address this question, we used fluorescent *in situ* hybridization to visualise the distribution of the poly(U)-tailed NA mRNA in infected cells (Fig. 7). We first used a riboprobe which is specific for the positive sense NA RNAs (i.e. detects both mRNA and cRNA) (Fig. 7, panels A to I). Since cRNAs are synthesized in low amounts compared to mRNAs during infection and they remain in the nucleus throughout viral infection, one may assume that any positive signal in the cytoplasm represents mRNAs, while a signal in the nucleus represents predominantly mRNA, although cRNA might partly contribute to this signal. As shown in Fig. 7 (panels D to F), cells infected with the wild-type virus showed strong signals in the cytoplasm. These results showed that poly(A)-tailed mRNAs were transported from the nucleus to the cytoplasm, as expected. By contrast, in cells infected with the A₆ mutant, positive sense NA RNAs could only be detected in the nucleus (Fig. 7, panels A to C), suggesting that poly(U)-mRNAs have a defect in nuclear transport and remain mainly in the nucleus. No signal was detected in mock infection (Fig 7, panels G to I).

To confirm that poly(U)-tailed NA mRNAs remained mainly in the nucleus after being synthesized, we used a riboprobe specific for the poly(U)-tailed NA mRNA, but not for NA cRNA, in the *in situ* hybridization. As expected; no poly(U)-tailed NA mRNA was detected in cells infected with wild-type virus (Fig: 7, panels M to O) or in mock infections (Fig. 7, panels P to R). By contrast, poly(U)-tailed NA mRNAs were detected in cells infected with the A₆ mutant (Fig. 7, panels J to L). More importantly, the poly(U)-tailed NA mRNA was predominantly detected in nuclei of infected cells (Fig 7, panels J to L). In summary, these results demonstrate that the poly(U)-tailed mRNA remains predominantly in the nucleus after synthesis, which strongly suggests that the poly(U)-tailed mRNA is defective in nuclear export.

### Example 7

### Pathogenicity and efficacy of the A6 mutant

The pathogenicity of and the protection provided by the A6 mutant was determined by using Balb/C mice. The mice were challenged with varying titres of wild-type or mutant virus to study pathogenicity and then observed for signs of pneumonia. To study the protective effect of the mutant virus mice infected with mutant influenza virus were subsequently challenged by infection with wild type influenza virus and monitored for illness. The mouse influenza challenge model is an established system for the study of influenza infection and has historically been used to determine the efficacy of vaccines or antiviral compounds against influenza.

42 female Balb/C mice (Charles River UK Ltd.) which were 6 to 12 weeks old and randomised by litter were used in the study. The mice were divided into 7 groups of 6 mice and were all electronically tagged to allow identification of each individual within a group. On day 0 all mice were anaesthetised using halothane/oxygen and infected with 50µl of influenza virus intranasally, the individual weight of each mouse was recorded. The seven groups of mice were administered with mutant or wild-type virus as shown in Table 1. All mice were individually observed daily for clinical symptoms and weighed. Any mice showing advanced symptoms of pneumonia or any mice losing 20% or more of their body weight were euthanased following a Home Office schedule 1 procedure. General health scoring of all mice was on a scale of 0 (full health) to 8. Any mice scored over a 7 were euthanased, and scored as an 8 for the rest of the study. The final weight of euthanased mice was included in the group mean for the remaining days of the study. All mice were observed for a period of 21 days post infection. Any surviving mice from the A/WSN/33 wild type groups were euthanased following a Home Office schedule 1 procedure. On Day 21-post infection, all remaining mice (including the uninfected control group) received a second infection of 50µl of A/WSN/33 wild type. All mice were observed daily for clinical symptoms. The same endpoints for euthanasia were used as before. After 10 days post second infection, the remaining mice were sacrificed. Table 2 shows the results of the pathogenicity study. Severe health effects were observed in mice infected with wild type virus on days or 2 or 3 onwards and most had to be euthanased. In contrast only slight effects were noticed on days 7 and 8 in a few mice of the group infected with the highest dose of mutant virus. No ill effects were observed at lower doses.

Table 3 shows the results of the protection study. The health and weight figures of challenged with wild type virus after initial infection with the poly U modified virus were significantly better than for the control group. Severe health effects and weight loss were observed in unprotected control group within 2 days of challenge. However, the health effects and weight loss were much less in mice which had been previously been infected with mutant virus at any dose, with almost no effects in the highest dose group. Thus the mutant virus provided significant protection against a dose of wild type virus that would normally be lethal.

### Discussion

The polyadenylation site in influenza virus vRNA templates has been mapped to a track of uridine residues near the 5' end of the vRNA. Here, we engineered an influenza virus which contains a U₆ to A₆ mutation at the 5' end of the NA vRNA segment. As a consequence of the U₆ to A₆ mutation, this novel transfectant virus synthesized NA mRNA with a poly(U)-tail instead of the usual poly(A) tail. It is not surprising that we did not detect any poly(A) sequence in the NA mRNA, since the poly(U)-tailed mRNA lacked the viral signal for polyadenylation. Moreover, the mRNA does not contain signals for the eukaryotic polyadenylation machinery. Thus, it is unlikely that any known host enzyme could add a poly(A) sequence to the poly(U)-tailed NA mRNA. Interestingly, low expression of NA was detected in cells infected with the A₆ mutant, indicating that poly(U)-tailed NA mRNA could be translated. The U₆ to A₆ mutation is surprisingly stable during viral passages; no reverse mutation in the A₆ track was detected even after 10 viral passages. By contrast, when the U₆ track was mutated into a random sequence (TCTACG), no virus was rescued, suggesting that a homopolymeric-track at the polyadenylation site was essential for mRNA synthesis. These results were confirmed by the CAT reporter assay (Fig. 2), in which a U6→ TCTACG mutant showed no CAT expression.

The A₆ mutant was attenuated in cell culture. When we analyzed the NA activity of the mutant virus, it was only about 8% of the wild type and this correlates with a reduced level of NA expression in cells infected with the A₆ mutant. NA is known to promote the release of viral particles from the cell surface by removal of terminal sialic acid from both the cell surface and virus glycoproteins, and thus promote viral spread. Therefore, the attenuation of the A₆ mutant was most likely due to the reduced levels ofNA expression in cells.

We detected reduced levels of poly(U)-tailed NA mRNA in infected cells, suggesting that the low NA expression could be due to the low levels of poly(U)-tailed NA mRNAs. Since model vRNA templates with either a U₆ or A₆ track could synthesize similar amounts of mRNAs *in vitro* (1), it was unlikely that the mutated NA vRNA of the A₆ mutant was a less efficient template for the synthesis of poly(U)-tailed mRNA. The observed reduction of poly(U)-tailed NA mRNA levels was more likely due to the reduction of the NA vRNA levels. When we analyzed the steady state levels of vRNAs in infected cells, there was a 50% reduction of NA vRNA levels. It was shown that the nonconserved nucleotides at the 3' and 5' ends of vRNA play an important role in replication. It is possible that the U₆ to A₆ mutation, which resulted in changing the nonconserved sequences of cRNA and vRNA, could somehow down-regulate the replication process. However, the NA cRNA level of the A₆ mutant was not affected by the mutation. In wild-type virus infection, vRNA can be transported to the cytoplasm for packaging into progeny virions or used as a template for transcription and replication. It is possible that, in the case of the A₆ mutant infection, NA vRNAs which are normally used for mRNA synthesis or transported to the cytoplasm are recruited to NA cRNA synthesis so as to maintain sufficient levels of NA cRNA templates for NA vRNA synthesis. The severe reduction of NA activity of the A₆ mutant virus (8% of that of the wild type) did not correlate with the reduced NA mRNA level in cells infected with the A₆ mutant (50% of that of the wild type). This indicates that the reduced level of poly(U)-tailed mRNA might not be the only factor that affects NA expression. From the results of *in situ* hybridization (Fig. 7), we observed different localization patterns of poly(A)-tailed and poly(U)-tailed NA mRNAs. Poly(A)-tailed NA mRNAs were mainly distributed in cytoplasm. By contrast, poly(U)-tailed NA mRNAs were found predominantly in the nucleus. Since protein synthesis occurs in the cytoplasm, a defect in the transport of poly(U)-tailed NA mRNAs from nucleus to cytoplasm would lead to limited amounts of mRNAs for protein expression. Hence, the severe reduction of NA protein expression in infected cells was most likely caused by the retention of poly(U)-tailed mRNAs in the nucleus. We were not able to detect NA mRNA in the cytoplasm of A₆ mutant-infected cells (Fig. 7) indicating that poly(U)-tailed NA mRNA was present in the cytoplasm at levels below our detection limit. On the other hand, the detection of NA protein in infected cells (Fig. 4B) and in the A₆ mutant virus (Fig. 4C) clearly demonstrated that a certain amount of poly(U)-tailed NA mRNAs was available for translation. Thus it had to be transported (or diffused) into the cytoplasm.

We do not know whether replacing the poly(A) tail of the NA mRNA with a poly(U) tail affects translation efficiency. However, knowing that the majority of the poly(U)-tailed mRNA remains in the nucleus, the significant level of NA protein expression (8% of that of the wild type) in the A6 mutant-infected cells suggests that the poly(U)-tailed NA mRNA is a competent template for translation. Non-polyadenylated influenza virus mRNAs are known to be translated under *in vitro* conditions. Results from the present study also show that polyadenylation is not required for translation. Furthermore, our results imply that other factors are involved in the control of viral translation. Thus, the 5' untranslated region of the viral mRNA, the NS 1 protein and some cellular proteins are known to facilitate the translation of viral mRNAs. Hence, the poly(A) tail of the influenza virus mRNA is not essential for translation and its most important function might be to direct influenza mRNA export from the nucleus to the cytoplasm.

Several influenza virus proteins have been shown to be involved in processes related to RNA transport. For example, nucleoprotein and matrix protein 1 are known to bind to vRNA and facilitate its nuclear export. Recently, NS2 (also known as NEP) was identified as having a similar function to the Rev protein of HIV-1 in mediating viral RNP export. However, these proteins seem to be restricted to mediating nuclear export of vRNA only. The NS 1 protein appears to interact with the cellular 3'-end processing machinery and inhibits the nuclear export of cellular poly(A)-containing mRNAs. However, new evidence suggests that these functions of NS 1 are dispensable for viral replication under certain conditions. Since influenza virus mRNAs have a similar structure to cellular mRNAs [e.g. 5' cap and poly(A) tail], it would not be surprising if they utilized cellular mRNA transport system for nuclear export.

In eukaryotic cells, there is a growing body of evidence supporting the idea that maturation of cellular pre-mRNAs is a prerequisite for nuclear export. In agreement with this idea, the addition of poly(A) tail was found to stimulate mRNA export. For non-polyadenylated histone mRNAs, the generation of mature 3' end is also known to be required for nuclear export . Here, in the absence of a characteristic signal of mature mRNA [e.g. poly(A) tail], cellular nuclear export factors might regard the poly(U)-tailed NA mRNA of the A₆ mutant as a pre-mRNA and retain the poly(U)-tailed mRNA in the nucleus. Since the synthesis of the poly(A) tail by the influenza virus RNA polymerase does not require the cellular cleavage/polyadenylation machinery, our results suggest that a poly(A) sequence itself is required for efficient mRNA export.

On the other hand, it is possible that the poly(U) tail, itself, is a signal for nuclear retention. In eukaryotic cells, several cellular proteins that are associated with the mRNA in the nucleus are selectively removed prior to mRNA nuclear export, indicating that some of these cellular proteins might be involved in nuclear retention. Interestingly, hnRNP C protein, a protein which is involved in mRNA nuclear retention, has been shown to have avid binding properties for a poly(U) sequence. We speculate that hnRNP C protein, or perhaps other nuclear retention factors (cellular and viral), might specifically bind to the poly(U) sequence of the poly(U)-tailed mRNA and impede the export of poly(U)-tailed mRNA from the nucleus to the cytoplasm.

### Materials and Methods

**Viruses.** Influenza A/WSN/HK virus, a reassortant virus containing an NA segment from influenza A/HK/8/68 virus and all other segments from influenza A/WSN/33 virus, was grown in embryonic chicken eggs. Influenza X-31 virus is a reassortant virus of influenza A/HK/8/68 virus and influenza A/PR/8/34.
**Plasmid transfection and CAT assay.** Plasmids pGT-h-PB 1, pGT-h-PB2, pGT-h-PA, pGT-h-NP, which express the influenza PB1, PB2, PA and NP proteins, respectively, and pPOLI-CAT-RT were generously provided by P. Palese (1, 19). One µg of each plasmid was transfected into 293 cells with 25 µl of DOTAP transfection reagent (Boehringer Mannheim) as instructed by the manufacturer. Mutated versions of the pPOLI-CAT-RT plasmid were made by an inverse PCR technique with *Pfu* DNA polymerase and the mutated sequences were confirmed by DNA sequencing. At about 60 hours post-transfection, cell extracts were harvested and tested for CAT activity as described (23). 50 µl of undiluted or diluted cell extracts were incubated in 75 µl reaction mixtures containing 0.08 µCi [¹⁴C]chloramphenicol, 1 mM acetyl-CoA and 250 mM Tris-HCl (pH 7.5). After incubation at 37°C for 2 hours, reaction products were extracted by ethyl acetate and then separated by thin-layer chromatography. Acetylated products were detected by autoradiography and analyzed in a phosphorimager.
**Preparation of influenza A virus RNP and RNP transfection.** RNA polymerase and nucleoprotein were isolated from influenza A virus, strain X-31, as described previously (25). Briefly, the virus was disrupted with Triton X-100, NP-40 and lysolecithin. RNP was separated by glycerol step gradient centrifugation. Fractions enriched in RNP were treated with micrococcal nuclease and then used, after the addition of EGTA, in RNP transfection. Plasmid pT3NAMI (5), which encodes thefull-length wild-typeNAvRNA segment of influenza A/WSN/33, was generously provided by P. Palese. Mutated versions of the plasmid were made by an inverse PCR technique with *Pfu* DNA polymerase and the mutated sequences were confirmed by DNA sequencing. RNP transfections were conducted as described (8). Briefly, *Bpu*AI-linearized pT3NAM 1 was transcribed by T3 RNA polymerase at 37°C for 30 min in the presence of micrococcal nuclease-treated RNP. The reconstituted RNP complexes were transfected with DEAE-dextran into MDBK cells infected with influenza A/WSN/HK helper virus. After a one day incubation, medium from transfected cells was checked for the presence of transfectant viruses by plaque assay on MDBK cells.
**Virus Purification.** Transfectant viruses were plaque purified three times in MDBK cells covered with agar overlay medium. Purified viruses were then grown in MDBK cells and purified through a 30% sucrose cushion, followed by purification on a 30 to 60% sucrose gradient.
**Sequencing of the 5' and 3' ends of the NA gene.** cDNA clones of the 5' end of the NA segment from transfectant viruses were obtained by rapid amplification of 5' DNA ends (5' RACE). NA vRNA from the purified transfectant viruses was reverse transcribed by using a primer 5'-GGGTGTCCTTCGACCAAAAC-3' (complementary to nt 879-898 of the NA vRNA) and SuperScript II reverse transcriptase (Gibco BRL). The reverse transcribed product was tailed with a poly(C) sequence at its 3' end by terminal deoxynucleotidyltransferase. The poly(C)-tailed cDNAs were amplified by PCR with a primer 5'-TGGACTAGTGGGAGCATCAT-3' (complementary to nt 1280-1299 of the NA vRNA) and a 5' RACE abridged anchor primer as instructed by the manufacturer (Gibco BRL). PCR products were then cloned into pGEM®-T vector (Promega) and sequenced. To synthesize cDNA of the 3' end sequence of the NA segment, vRNAs from purified viruses were first polyadenylated by poly(A) polymerase (Gibco BRL). The polyadenylated vRNA was reverse transcribed by using a primer 5'-GCGCTCTAGAATTCTTTTTTTTTTTTTTTTAGC-3' cDNA was amplified by PCR with the primer for reverse transcription and a primer 5'-GTTGAGTCCTGCCCAGCAACAACT-3' corresponding to nt 1197-1220 of the NA vRNA. PCR products were then cloned into pGEM®-T and sequenced.
**NA activity assay.** The NA activity was determined as described before (25). Briefly, purified viruses (∼ 3 µg) were incubated in 100 µl 150 mM phosphate buffer containing 1 mM CaCl₂ and 50 nmol of 2'-(4-methyl-umbelliferyl)-α-D-*N*-acetylneuraminic acid (MU-NANA) (Sigma) for 10 min at 37°C. Reactions were then stopped by adding 2 ml of 0.5 M glycine-NaOH (pH 10.4) and the released 4-methylumbelliferone was assayed by using a spectrofluorimeter (Shimadzu, RF-540). A 0.1 mM solution of 4-methylumbelliferone (Sigma) was used as a standard control.
**Immunofluorescence microscopy.** MDBK cells grown on glass cover slides were washed with phosphate buffered saline (PB S) and then infected with viruses at about 0.5 moi. Infected cells were harvested at 10 hr p.i., washed twice in PBS and fixed in 4% paraformaldehyde in PBS. Fixed cells were permeabilised with 0.5% Triton-X-100 in PBS for 15 min, followed by two washes in PBS. Cells were then incubated with a 100 times diluted mouse monoclonal anti-NA antibody (H17-L17-5; a generous gift from Walter Gerhard) overnight at 4°C. After two washes in PBS with 0.01% Triton-X-100, cells were incubated with a horse anti-mouse antibody conjugated with fluorescein (Vector; 1: 200 dilution) for one hour at room temperature. Samples were washed twice in PBS containing 0.01% Triton-X-100 and then mounted in antibleach medium containing 4',6-diamidino-2-phenylindole (DAPI) for DNA staining (Vector). Samples were examined on a Zeiss Axiophot microscope.
**RT-PCR assays for mRNA and vRNA from infected cells.** MDBK cells infected with viruses at an moi of 2 were harvested at 12 hr p.i. Total RNA was isolated with Trizol (Gibco BRL) as instructed by the manufacturer. To detect the NA vRNA, about 1.5 µg total RNA was first reverse transcribed by 25 U Moloney murine leukemia virus reverse transcriptase (M-MLV RT, Promega) at 42°C for 30 min in the presence of an NA vRNA-specific primer 5'-GGGTGTCCTTCGACCAAAAC-3' (complementary to nt 879-898 of the NA vRNA). The reverse transcribed products were then amplified by Taq polymerase with primers 5'-TGGACTAGTGGGAGCATCAT-3' (complementary to nt 1280-1299 of the NA vRNA) and 5' ggactagtagtAGTAGAAACAAGG-3' (underlined nt corresponding to the first 13 nt of the 5' end of NA vRNA).

To detect the homopolymeric tails of NA mRNA; RT-PCR was performed as described before (22). About 1.5 µg total RNA was first reverse transcribed by 25 U M-MLV RT (Promega) at 42°C for 30 min in the presence of either a 5' GC-clamped T₂₀ primer [5'-GCCCCGGGATCCT₂₀-3', specific for poly(A)-tailed mRNA] or a 5' GC-clamped A₂₀ primer [5'-GCCCCGGGATCCA₂₀-3', specific for poly(U)-tailed mRNA]. The reverse transcribed products were then amplified by Taq polymerase using 5'-TGGACTAGTGGGAGCATCAT-3' (complementary to nt 1280-1299 of the NA gene) and the corresponding primer specific for the homopolymeric tail. All PCR products were then cloned into pGEM®-T and sequenced.
**Primer extension assay.** Total RNA from cells infected with viruses at an moi of 2 was isolated with Trizol (Gibco BRL) at the indicated time points. Primer extension assays were performed as described before (25). About 0.2 µg of viral RNA or 5 µg of total RNA were reverse transcribed by 200 U SuperScript (Gibco BRL) for 45 min at 42°C in the presence of the corresponding ³²P-labelled primer. Reactions were terminated by adding equal volume of formamide loading dye. Products were then heated to 99°C for 2 min and analyzed on 5% polyacrylamide-7 M urea gels. To quantify the yield of the reverse transcribed products, gels were dried and analyzed in a Phosphorimager.

To determine NA and NS vRNA levels, an NA vRNA-specific primer (5'-GTGGCAATAACTAATCGGTCA-3', complementary to nt 1151-1171 of NA vRNA) and a NS vRNA-specific primer (5'-GGGAACAATTAGGTCAGAAGT-3', complementary to nt 695-715 of NS vRNA) were used in the primer extension assay. An NA cRNA/mRNA-specific primer (5'-GTTGAGTCCTGCCCAGCAACAACT-3', corresponding to nt 142-122 of the NA vRNA) and an HA cRNA/mRNA-specific primer (5'-CATATTGTGTCTGCATCTGTAGCT-3', corresponding to nt 94-71 of the HA vRNA) were used to determine mRNA and cRNA levels of NA and HA, respectively.
***In situ*** **hybridization.** MDBK cells grown on glass cover slips were washed with PBS and then infected with viruses at an moi of 0.5. Infected cells were harvested at 8 hour p.i., washed twice with PBS and fixed in 4% paraformaldehyde in PBS. The fixed cells were then permeablized by PBS containing 0.5% Triton X-100 for 15 min at room temperature. Cells were then washed twice in PB S followed by washing in 2xSSC (1X SSC = 0.15 M NaCl, 0.015 M sodium citrate) twice. Samples were hybridized in 20 µl of hybridization buffer (50% formamide, 2X SSC, 10% dextran sulphate, 1mg/ ml of *E. coli* tRNA) containing about 100 ng of riboprobe at 37°C for overnight. After hybridization, cells were washed twice in hybridization buffer at 37°C, followed by successive washings for 15 min with 2X SSC, 1X SSC, 0.5X SSC and 0.2X SSC at room temperature. Samples were then incubated in 4X SSC containing 1 µg/ml anti-digoxigenin-fluorescein antibody (Boehringer Mannheim) for I hour at room temperature. After the incubation, samples were washed with 4X SSC containing 0.01% Triton X-100 three times and mounted in antibleach medium containing DAPI for DNA staining (Vector). Samples were examined by a Zeiss Axiophot microscope.

For NA cRNA and NA mRNA detection, a riboprobe (corresponding to nt 1 to 299 of the NA vRNA) was prepared by T3 RNA polymerase transcription of *BamH*I-linearized pT3NAM1. For poly(U) tail mRNA detection, a plasmid which contains a poly(A) sequence and part of 3' end of the NA gene was constructed. The transcription product from this plasmid contained a sequence of 86 A residues [specific for the poly(U) tail] and the last 21 NA-specific residues of NA mRNA. Both riboprobes were labelled with digoxigenin-UTP (Boehringer Mannheim) during *in vitro* transcriptions.

### References

1. **Poon, L. L. M., D. C. Pritlove, E. Fodor, and G. G. Brownlee.** 1999. Direct evidence that the poly(A) tail of influenza A virus mRNA is synthesis by reiterative copying of a U-track in the vRNA template. J. Virol. **73**:3473-3476.
2. **Muster T., Ferko B., Klima, A.** ***et al*****.** 1995. Mucosal model of immunisation against human immunodeficiency virus type 1 with a chimeric influenza virus. J. Virol. 69, 6678-86.
3. **Li, S., Polords, V., Isobe, H.** ***et al*****.** 1993. Chimeric influenza virus induces neutralising antibodies and cytotoxic **T** cells against human immunodeficiency virus type 1. J. Virol. 67, 6659-66.
4. **Rodrigues, M., Li, S., Murata, K., Rodrigues D.** 1994. Influenza and vaccinia viruses expressing malaria CD8+T and B cell epitopes. J. Immunol. 153, 4636-48.
5. **Garcia-Sastre, A., T. Muster, W. S. Barclay, N. Percy, and P. Palese.** 1994. Use of a mammalian internal ribosomal entry site element for expression of a foreign protein by a transfectant influenza virus. J. Virol. 68: 6254-6261.
6. **Percy, N., Barclay, W. S., Garcia-Sastre, A. and Palese, P.** 1994. Expression of a foreign protein by influenza A virus. J. Virol 68, 4486-92.
7. **Enami, M., W. Luytjes, M. Krystal, and P. Palese.** 1990. Introduction of site specific mutations into the genome of influenza virus. Proc. Natl. Acad. Sci. USA 87: 3802-3805.
8. **Enami, M., and P. Palese.** 1991. High-efficiency formation of influenza virus transfectants. J. Virol. 65: 2711-2713.
9. **Garcia-Sastre, A. and Palese, P.** 1993. Genetic manipulation of negative-strand RNA virus genomes. Ann. Rev. Microbiol. 47, 765-90.
10. **Luytjes, W., M. Krystal, M. Enami, J. D. Parvin, and P. Palese.** 1989. Amplification, expression, and packaging of a foreign gene by influenza virus. Cell. 59: 1107-1113.
11. **Li, S., Xu, M. and Coelingh, K.** 1995. Electroporation of ribonucleoprotein complexes for rescue of the nucleoprotein and matrix genes. Virus Res. 37, 153-161.
12. **Muster, T. and García-Sastre, M.** June 1998. Textbook of Influenza, Blackwell Science Ch. 9, p.93-106, Genetic Manipulation of Influenza Viruses.
13. **Liu, C. and Air, G. M.** 1993. Selection and characterisation of a neuraminidase-minus mutant of influenza virus and its rescue by cloned neuraminidase genes. Virology 194,403-7.
14. **Enami, M., Sharma, G., Benham, G. and Palese, P.** 1991. An influenza virus containing nine different RNA segments. Virology 185, 291-8.
15. **Subbarao, E. K., Park, E.J., Lawson, C.M., Chen, A.Y. and Murphy, B.R.** 1995. Sequential addition of temperature-sensitive missense mutations into the PB2 gene of influenza A transfectant virus can effect an increase in temperature sensitivity and attenuation and permits the rational design of a genetically engineered live influenza A virus vaccine J. Virol. 69, 5969-77.
16. **Castrucci M. R. and Kawaoka, Y.** 1995. Reverse genetics system for generation of an influenza A virus mutant containing a deletion of the carboxyl-terminal residue of M2 protein. J. Virol. 69, 2725-8.
17. **Horimoto, T. and Kawaoka, Y.** 1994. Reverse genetics provides direct evidence for a correlation of haemagglutinin cleavability and virulence of an avian influenza A virus. J. Virol. 68, 3120-3128.
18. **Barclay, W.S. and Palese, P.** 1995. Influenza B viruses with site-specific mutations introduced into the NA gene. J. Virol. 76, 3211-5.
19. **Pleschka, S., Jaskunas, R., Engelhardt, O.G., Zurcher, T., Palese P. and García-Sastre, A.** 1996. A plasmid-based reverse genetics system for influenza A virus. J. Virol. 70, 4188-92.
20. **Enami, M.** 1997. Improved technique to genetically manipulate influenza virus. In Frontiers ofRNA Virus Research p.19; The Oji International Seminar in Natural Science, Kyoto, Japan 1997.
21. **Ferko, B., Egorov, A.** ***et al*****.** 1997. Influenza virus as a vector for mucosal immunisation. In Frontiers of RNA Virus Research, p.18. The Oji International Seminar in Natural Science, Kyoto, Japan.
22. **Pritlove, D. C., L. L. M. Poon, E. Fodor, J. Sharps, and G. G. Brownlee.** 1998. Polyadenylation of influenza virus mRNA transcribed *in vitro* from model virion RNA templates: requirement for 5' conserved sequences. J. Virol. **72**:1280-1286.
23. **Gorman, M., L. F. Moffat, and B. H. Howard.** 1977. Recombinant genomes which express chloramphenicol acetyl-transferase in mammalian cell. Mol. Cell. Biol. **2**:1044-1051.
24. **Seong, B. L., and G. G. Brownlee.** 1992. A new method for reconstituting influenza polymerase and RNA *in vitro*: a study of the promoter elements for cRNA and vRNA synthesis *in vitro* and viral rescue *in vivo.* Virology **186:**247-260.
25. **Fodor, E., P. Palese, G. G. Brownlee, and A. Garcia-Sastre.** 1998. Attenuation of influenza A virus mRNA levels by promoter mutations. J. Virol. **72**:6283-6290.
26. **Egorov, A. et al.** 1998. Transfectant influenza A viruses with long deletions in the NS 1 protein grow efficiently in Vero cells. J. Virol. **72**:6437-6441.
27. **Fodor et al.** 1999. Rescue of Influenza A virus from Recombinant DNA. J. Virol. 73:9679-9682.
28. **Neumann et al.** 1999. Generation of influenza A viruses entirely from cloned cDNAs. Proc. Natl. Acad. Sci. USA. 96: 9345-9350.
29. **Zeng et al.** 1996. Nonconserved nucleotides at the 3' and 5' ends of an influenza A virus RNA play an important role in viral replication. Virology 217:242-251.

**Table 1.**

| Immunisation schedule | | |
|---|---|---|
| **Group** **No.** | **Antigen Day 0** | **Challenge Virus Day** **21** |
| 1 | A/WSN/33 wild type 1x10⁶ pfu | N.A. |
| 2 | A/WSN/33 wild type 3x10⁴ pfu | N.A. |
| 3 | A/WSN/33 wild type 1X10³ pfu | N.A. |
| 4 | A/WSN/33 A6 mutant 1x10⁶ pfu | A/WSN/33 wild type 1x10⁶ pfu |
| 5 | A/WSN/33 A6 mutant 3x10⁴ pfu | A/WSN/33 wild type 1x10⁶ pfu |
| 6 | A/WSN/33 A6 mutant 1X10³ pfu | A/WSN/33 wild type 1x10⁶ pfu |
| 7 | No Antigen (PBS) | A/WSN/33 wild type 1X10⁶ pfu |

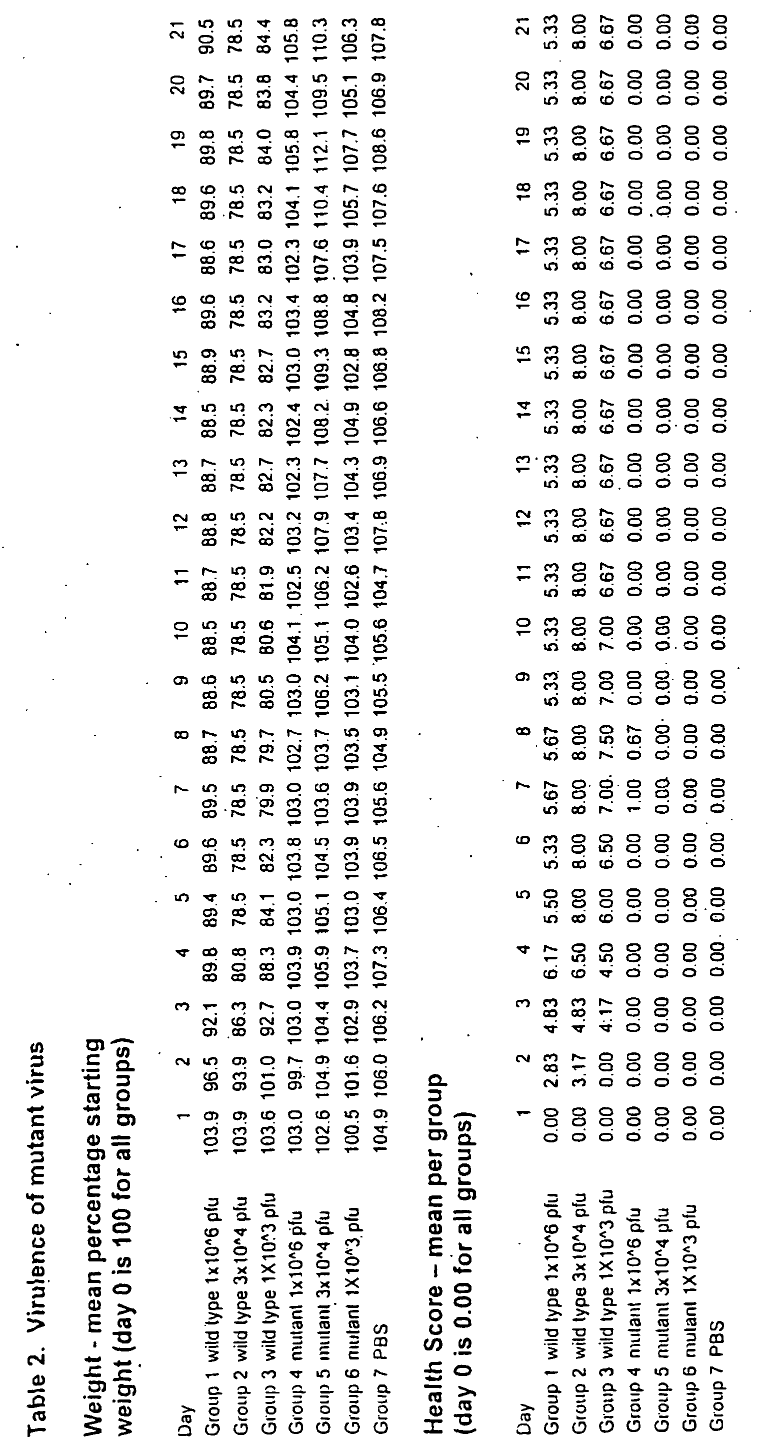

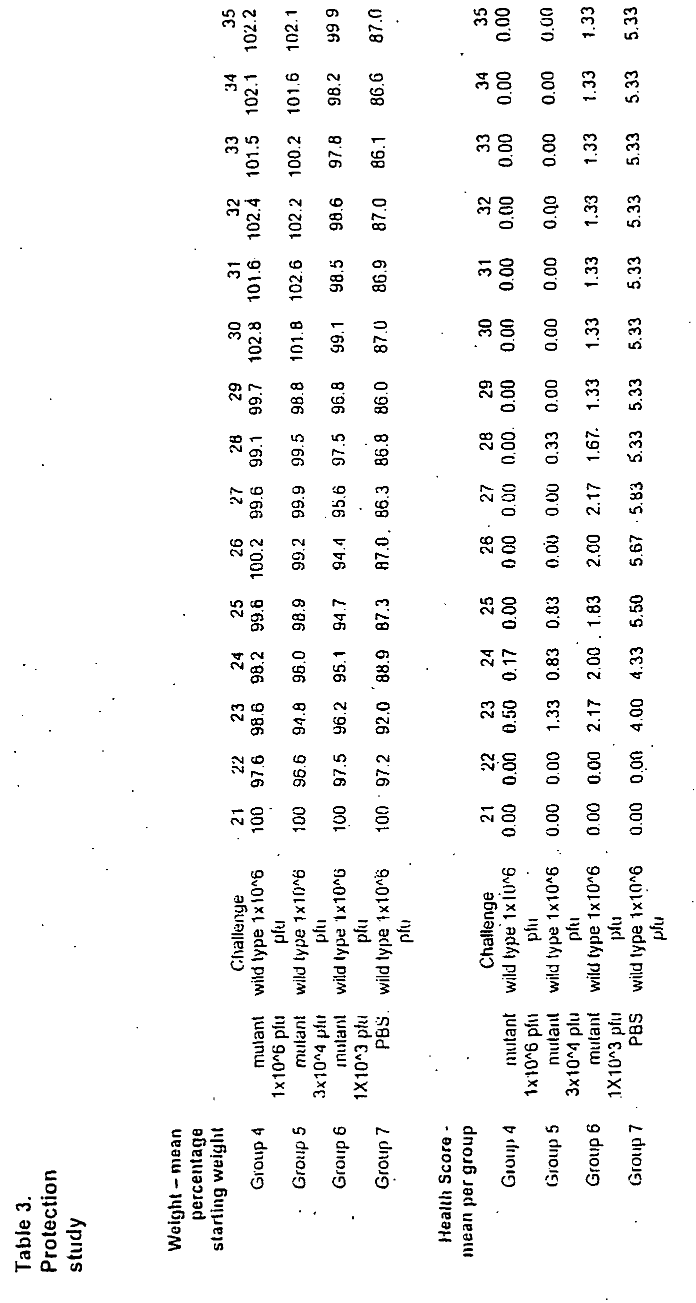

## Claims

1. An attenuated negative-sense single stranded influenza virus comprising genomic RNA segments, at least one of which is altered by replacement of the poly U track thereof, located at the polyadenylation site near the 5'-end of the genomic RNA, by a poly A track which is capable of being copied to provide a 3'-poly U tail for mRNA transcribed from the genomic RNA segment.

2. A virus according to Claim 1, which is an influenza A, B or C virus.

3. A virus according to Claim 1 or 2, in which the genomic RNA segment altered by replacement of the poly U track encodes a neuraminidase.

4. A virus according to Claim 1, 2 or 3, in which the nucleic acid is a mutated native influenza virus genomic RNA segment.

5. A virus according to any one of Claims 1 to 4, which exhibits at least a 0.5 log reduction in plaque titre compared to the parent wild type virus on MDCK cells.

6. A virus according to any one of the preceding Claims, which additionally comprises a heterologous coding sequence capable of being expressed in target cells.

7. A virus according to Claim 6, wherein said heterologous coding sequence encodes an antigenic peptide or polypeptide capable of stimulating an immune response to a pathogenic agent.

8. A ribonucleoprotein (RNP) complex wherein a nucleic acid is complexed with polymerase proteins and nucleoprotein of an influenza virus for use in preparing an attenuated virus as claimed in any one of Claims 1 to 7, wherein the nucleic acid comprises a genomic RNA segment of an attenuated negative-sense single stranded influenza virus altered by replacement of the poly U track thereof, located at the polyadenylation site near the 5'-end of the genomic RNA, by a poly A track which is capable of being copied to provide a 3'-poly U tail for mRNA transcribed from the genomic RNA segment.

9. An *ex vivo* cell infected by a virus as defined in any one of Claims 1 to 7.

10. A vaccine comprising a virus as claimed in any one of Claims 1 to 7.

11. A vaccine as claimed in Claim 10, which comprises a virus as defined in Claim 7 and which is capable of stimulating an immune response to an influenza virus and a second pathogenic agent other than an influenza virus.

12. A pharmaceutical composition comprising a virus as defined in Claim 6 or 7, in combination with a pharmaceutically acceptable carrier or diluent for delivery of said heterologous coding sequence to target cells.

13. A pharmaceutical composition comprising cells infected with a virus according to Claim 6 or 7, in combination with a pharmaceutically acceptable carrier or diluent.

14. A method of preparing a virus according to any one of Claims 1 to 7, which comprises providing in a host cell the genomic nucleic acid segments for said virus under conditions whereby said segments are packaged into a viral particle, wherein at least one of the segments is a nucleic acid that comprises a genomic RNA segment of an attenuated negative-sense single stranded influenza virus altered by replacement of the poly U track thereof, located at the polyadenylation site near the 5'-end of the genomic RNA, by a poly A track which is capable of being copied to provide a 3'-poly U tail for mRNA transcribed from the genomic RNA segment.

15. Use of a virus as claimed in any one of Claims 1 to 7, as a helper virus to rescue an influenza virus genomic nucleic acid segment in cells, wherein viruses produced containing said nucleic acid segment are selected on the basis of increased growth, compared with the helper virus, on cells of a selected type.

16. A virus as claimed in any one of Claims 1 to 5, for administration to a patient to stimulate an immune response against an influenza virus.

17. A method of delivering a heterologous coding sequence *ex vivo* to cells which comprises infecting said cells *ex vivo* with a virus according to Claim 6 or 7 carrying said sequence.

## Patentansprüche

1. Attenuiertes einzelsträngiges Negativ-Sinn-Influenza-Virus, umfassend genomische RNA-Segmente, von denen mindestens eines verändert ist durch Ersetzung seines Poly-U-Abschnitts, der an der Polyadenylierungs-Stelle nahe dem 5'-Ende der genomischen RNA liegt, durch einen Poly-A-Abschnitt, der in der Lage ist, kopiert zu werden, um einen 3'-Poly-U-Schwanz für von dem genomischen RNA-Segment transkribierte mRNA vorzusehen.

2. Virus gemäß Anspruch 1, bei dem es sich um ein Influenza-A-, -B- oder -C-Virus handelt.

3. Virus gemäß Anspruch 1 oder 2, in welchem das genomische RNA-Segment, das durch Ersetzen des Poly-U-Abschnitts verändert ist, eine Neuraminidase codiert.

4. Virus gemäß Anspruch 1, 2 oder 3, in welchem die Nukleinsäure ein genomisches mutiertes natives Influenza-Virus-RNA-Segment ist.

5. Virus gemäß mindestens einem der Ansprüche 1 bis 4, welches mindestens eine Reduktion um 0,5 log im Plaque-Titer im Vergleich zum Eltern-Wildtyp-Virus auf MDCK-Zellen aufweist.

6. Virus gemäß mindestens einem der vorangehenden Ansprüche, welches zusätzlich eine heterologe codierende Sequenz umfasst, die in der Lage ist, in Zielzellen exprimiert zu werden.

7. Virus gemäß Anspruch 6, wobei die heterologe codierende Sequenz ein antigenes Peptid oder Polypeptid codiert, das in der Lage ist, eine Immunantwort gegen ein pathogenes Agens zu stimulieren.

8. Ribonukleoprotein(RNP)-Komplex, worin eine Nukleinsäure mit Polymerase-Proteinen und Nukleoprotein eines Influenza-Virus komplexiert ist, zur Verwendung bei der Herstellung eines attenuierten Virus, wie beansprucht in einem beliebigen der Ansprüche 1 bis 7, wobei die Nukleinsäure ein genomisches RNA-Segment eines attenuierten einzelsträngigen Negativ-Sinn-Influenza-Virus umfasst, das verändert ist durch Ersetzung von dessen Poly-U-Abschnitt, der an der Polyadenylierungs-Stelle nahe dem 5'-Ende der genomischen RNA liegt, durch einen Poly-A-Abschnitt, der in der Lage ist, kopiert zu werden, um einen 3'-Poly-U-Schwanz für von dem genomischen RNA-Segment transkribierte m RNA vorzusehen.

9. Zelle *ex vivo*, welche durch ein Virus infiziert ist, wie definiert in einem beliebigen der Ansprüche 1 bis 7.

10. Impfstoff, umfassend ein Virus, wie beansprucht in einem beliebigen der Ansprüche 1 bis 7.

11. Impfstoff, wie beansprucht in Anspruch 10, welcher ein Virus umfasst, wie definiert in Anspruch 7, und welcher in der Lage ist, eine Immunantwort gegen ein Influenza-Virus und ein zweites pathogenes Agens, das von einem Influenza-Virus verschieden ist, zu stimulieren.

12. Pharmazeutische Zusammensetzung, umfassend ein Virus, wie definiert in Anspruch 6 oder 7, in Kombination mit einem pharmazeutisch annehmbaren Träger oder Verdünnungsmittel zur Zuführung der heterologen codierenden Sequenz an Zielzellen.

13. Pharmazeutische Zusammensetzung, umfassend Zellen, die mit einem Virus gemäß Anspruch 6 oder 7 infiziert sind, in Kombination mit einem pharmazeutisch annehmbaren Träger oder Verdünnungsmittel.

14. Verfahren zur Herstellung eines Virus gemäß mindestens einem der Ansprüche 1 bis 7, welches das Vorsehen, in einer Wirtszelle, der genomischen Nukleinsäuresegmente für das Virus unter Bedingungen umfasst, bei welchen die Segmente in ein Viruspartikel verpackt werden, wobei mindestens eines der Segmente eine Nukleinsäure ist, die ein genomisches RNA-Segment eines attenuierten einzelsträngigen Negativ-Sinn-Influenza-Virus umfasst, das verändert ist durch Ersetzung von dessen Poly-U-Abschnitt, der an der Polyadenylierungs-Stelle nahe dem 5'-Ende der genomischen RNA liegt, durch einen Poly-A-Abschnitt, der in der Lage ist, kopiert zu werden, um einen 3'-Poly-U-Schwanz für von dem genomischen RNA-Segment transkribierte mRNA vorzusehen.

15. Verwendung eines Virus, wie beansprucht in einem beliebigen der Ansprüche 1 bis 7, als ein Helfervirus zum Retten eines genomischen Influenza-Virus-Nukleinsäuresegments in Zellen, wobei das Nukleinsäuresegment enthaltende produzierte Viren auf der Basis des im Vergleich zu dem Helfervirus erhöhten Wachstums auf Zellen eines gewählten Typs selektiert werden.

16. Virus, wie beansprucht in einem beliebigen der Ansprüche 1 bis 5, zur Verabreichung an einen Patienten, um eine Immunantwort gegen ein Influenza-Virus zu stimulieren.

17. Verfahren zur Zuführung einer hoterologen codierenden Sequenz *ex vivo* an Zellen, welches das Infizieren der Zellen *ex vivo* mit einem Virus gemäß Anspruch 6 oder 7, das die Sequenz trägt, umfasst.

## Revendications

1. Virus de la grippe atténuée, simple brin de sens négatif, comprenant des segments d'ARN génomique, dont au moins l'un est altéré par remplacement de sa queue poly U, située sur le site de polyadénylation au voisinage de l'extrémité 5' de l'ARN génomique, par une queue poly A qui est capable d'être copiée pour donner une queue 3'-poly U pour l'ARNm transcrit à partir du segment d'ARN génomique.

2. Virus selon la revendication 1, qui est un virus de la grippe A, B ou C.

3. Virus selon la revendication 1 ou 2, dans lequel le segment d'ARN génomique altéré par remplacement de la queue poly U code pour une neuraminidase.

4. Virus selon la revendication 1, 2 ou 3, dans lequel l'acide nucléique est un segment d'ARN génomique de virus natif muté de la grippe.

5. Virus selon l'une quelconque des revendications 1 à 4, qui présente une réduction d'au moins 0,5 log du titre des plages par comparaison avec le virus parent de type sauvage sur des cellules MDCK.

6. Virus selon l'une quelconque des revendications précédentes, qui comprend en outre une séquence codante hétérologue, capable d'être exprimée dans des cellules cibles.

7. Virus selon la revendication 6, dans lequel ladite séquence codante hétérologue code pour un peptide ou un polypeptide antigénique capable de stimuler une réponse immune à un agent pathogène.

8. Complexe de ribonucléoprotéines (RNP), dans lequel un acide nucléique est complexé avec des protéines de polymérase et une nucléoprotéine d'un virus de la grippe pour utilisation dans la préparation d'un virus atténué selon l'une quelconque des revendications 1 à 7, où l'acide nucléique comprend un segment d'ARN génomique d'un virus de la grippe simple brin de sens négatif atténué, altéré par remplacement de sa queue poly U, située sur le site de polyadénylation au voisinage de l'extrémité 5' de l'ARN génomique, par une queue poly A qui est capable d'être copiée pour donner une queue 3'-poly U pour l'ARNm transcrit à partir du segment d'ARN génomique.

9. Cellule *ex vivo* infectée par un virus selon l'une quelconque des revendications 1 à 7.

10. Vaccin comprenant un virus selon l'une quelconque des revendications 1 à 7.

11. Vaccin selon la revendication 10, qui comprend un virus selon la revendication 7 et qui est capable de stimuler une réponse immune à un virus de la grippe et à un deuxième agent pathogène autre qu'un virus de la grippe.

12. Composition pharmaceutique comprenant un virus selon la revendication 6 ou 7, en combinaison avec un excipient ou diluant acceptable d'un point de vue pharmaceutique, pour fourniture de ladite séquence codante hétérologue à des cellules cibles.

13. Composition pharmaceutique comprenant des cellules infectées par un virus selon la revendication 6 ou 7, en combinaison avec un excipient ou diluant acceptable d'un point de vue pharmaceutique.

14. Procédé de préparation d'un virus selon l'une quelconque des revendications 1 à 7, qui comprend la fourniture, dans une cellule hôte, des segments d'acide nucléique génomique pour ledit virus dans des conditions dans lesquelles lesdits segments sont encapsidés dans une particule virale, où au moins l'un des segments est un acide nucléique qui comprend un segment d'ARN génomique d'un virus de la grippe simple brin de sens négatif atténué, altéré par remplacement de sa queue poly U, située au niveau du site de polyadénylation au voisinage de l'extrémité 5' de l'ARN génomique, par une queue poly A qui est capable d'être copiée pour fournir une queue 3'-poly U pour l'ARNm transcrit à partir du segment d'ARN génomique.

15. Utilisation d'un virus selon l'une quelconque des revendications 1 à 7 en tant que virus assistant pour sauvegarder un segment d'acide nucléique génomique du virus de la grippe dans des cellules, pour laquelle les virus produits, contenant ledit segment d'acide nucléique, sont sélectionnés sur la base d'une augmentation de leur croissance par comparaison avec celle du virus assistant, sur des cellules d'un type sélectionné.

16. Virus selon l'une quelconque des revendications 1 à 5, pour administration à un patient pour stimuler une réponse immune à l'encontre d'un virus de la grippe.

17. Procédé pour amener une séquence codante hétérologue *ex vivo* à des cellules, qui comprend l'infection desdites cellules *ex vivo* avec un virus selon la revendication 6 ou 7 et portant ladite séquence.
